# EUROPEAN PATENT APPLICATION

(11) **EP 4 413 972 A1**
(43) Date of publication of application: **14.08.2024**
(21) Application number: 23156180.4
(22) Date of filing: 10.02.2023
(51) Int. Cl.: A61K 8/42, A61K 8/60, A61K 8/27, A61Q 19/00

(54) **A PRESERVATIVE-FREE SELF-PRESERVING COMPOSITION FOR TOPICAL APPLICATION**

(71) Applicant: Skinome Research AB, 117 28 Stockholm (SE)
(72) Inventor: ÅKERSTRÖM, Ulf, 117 28 STOCKHOLM (SE); GILLBRO, Johanna, 117 28 STOCKHOLM (SE); LATASTE, Sylvain, 117 28 STOCKHOLM (SE)
(74) Representative: Brann AB

(57) **Abstract**

A composition for topical application free of conventional preservatives and having antimicrobial properties comprising a combination of 0.1-2.0% by weight of urea, 0.1-2.0% by weight of lactobionic acid, 0.05-0.2% by weight of zinc; and 20-90% by weight of water of the total weight of the composition is disclosed, which compositions can be used for cosmetic, pharmaceutical and medical device applications.

## Description

### FIELD OF THE INVENTION

The present invention relates to compositions for topical application free of conventional preservatives and having antimicrobial properties, and more particularly to such compositions including a specific combination of compounds, viz. urea, lactobionic acid, and zinc, in certain amounts.

### BACKGROUND ART

In compositions for topical application, especially in aqueous compositions, e.g. containing 20 % by weight or more of water, one or more preservatives are typically included in order to provide a sufficient shelf-life of the product and to avoid microbial growth in the product due to contamination during manufacturing, storage, and use by the consumers during shelf-life of the product. The amount of preservative(s) used when intentionally added in the prior art may vary depending on the specific preservative(s), but generally ranges from an overall amount of 0.01 % to 1.5 % by weight of the composition.

The problem of undesired microbial growth in a topical product generally increases with an increased water content.

The presence in topical compositions of such conventionally used preservatives may however be undesirable for various reasons. Preservatives are one of the main causes of skin irritation and contact allergies, they are well-known as skin sensitizers, and this is the reason why they are associated with contact allergy, irritation and urticaria. Preservatives are considered a major health concern. Preservatives can disturb the skin-barrier function and influence the natural skin microbiome negatively, which in the end can lead to an imbalanced skin and skin microbiome, consequently promoting skin disorders like atopic dermatitis, rosacea, psoriasis, and seborrhoeic eczema. Preservatives can also be hazardous to environment, especially if they are persistent to degradation, bioaccumulating, and/or highly toxic to aquatic organisms. Preservatives therefore also pose an environmental risk.

It is an object of the present invention to provide a composition for topical application not requiring the presence therein of a conventional preservative.

### SUMMARY OF THE INVENTION

The above object has been accomplished by means of a composition comprising a combination of urea, lactobionic acid, and zinc, in specific amounts.

Accordingly, in one aspect, the present invention relates to a composition for topical application, comprising the following components: 0.1-2.0 % by weight of urea; 0.1-2.0 % by weight of lactobionic acid; 0.05-0.2 % by weight of Zn; and 20-90 % by weight of water of the total weight of the composition.

In the composition, the zinc is present in the form of Zn²⁺, and is obtained from a water soluble zinc salt from which the composition is formed.

Each one of the components of the inventive composition is known in the art to have a skin caring effect.

The inventive composition is believed to reduce the risk of negatively affecting the microbiome of the skin.

The composition does not include a conventional preservative.

The inventive composition can be formulated for different applications within wide ranges, both for cosmetical use, and for pharmaceutical application, and for medical device applications.

The inventive composition has a pH value which is compatible with the skin, preferably within a range of 3 to 9, more preferably within a range of 4 to 8, more preferably within a range of 4.5 to 7, and especially preferred within a range of 5 to 6.

Further embodiments and advantages of the invention will be apparent from the following detailed description and appended claims.

As used herein, the term "preservative-free" is intended to refer to the absence of an intentionally added conventional preservative. Preferably, also the ingredients used to prepare the inventive composition should be preservative-free.

### DETAILED DESCRIPTION OF THE INVENTION

The present inventors have surprisingly found that a combination of urea, lactobionic acid, and zinc in the form of Zn²⁺ ions, in relatively low and skin friendly amounts will provide an antimicrobial effect in an aqueous composition for topical application in the absence of conventional preservatives. Advantageously, each one of the components of the inventive composition is known in the art to have a skin caring effect. The antimicrobial effect of the inventive composition is sufficiently powerful to meet the requirements placed on cosmetics and topical drugs, respectively, in the absence of a conventional preservative in the composition. It is believed that a composition for topical application, which is free of conventional preservatives, will generally stand a better chance of allowing for retaining a balanced microbiome on an area of skin to which the composition has been applied. Accordingly, the inventive composition is believed to enable a more balanced and diversified microbiome, when applied to the skin. The invention also reduces the risk for side-effects or negative skin reactions which may be caused by conventional preservatives.

The inventive compositions have sufficiently powerful antimicrobial effect to meet the requirements placed on cosmetics and/or topical drugs, e.g. as required according to European Pharmacopeia 11.0. 5.1.3 Efficacy of antimicrobial preservation, topical preparations. By virtue of fulfilling the above requirements, the inventive compositions are also referred to herein as being "self-preserving".

As will be shown in the examples, compositions comprising 0,16% Zn²⁺ + 0,5% urea + 1% lactobionic acid, and 0,10% Zn²⁺ + 0,7% urea + 1,0% lactobionic acid has been found sufficiently powerful antimicrobial effect to meet the requirements placed on cosmetics and/or topical drugs. It is believed that a lower amount of any one, or two, of the three actives can be compensated by a higher amount of the remaining two, or one, respectively, within reasonable limits, provided that the combined amount of Zn, urea, and lactobionic acid is at least 1.8 % by weight. Preliminary results, yet to be confirmed, indicate that even merely 0.08 % by weight of Zn, 0.5 % by weight of urea, and 0.5 % by weight lactobionic acid, will provide sufficiently powerful antimicrobial effect to meet the requirements placed on cosmetics and/or topical drugs. Based thereupon it is believed that a composition within the above ranges comprising a total of at least 1.1 % by weight of Zn²⁺, urea, and lactobionic acid will provide sufficiently powerful antimicrobial effect to meet the requirements placed on cosmetics and/or topical drugs.

### Urea

Urea is a well-known moisturizer, which is part of skin own Natural Moisturising Factors (NMF). Urea is preferably included in an amount within the range of 0.5% to 2%, more preferably within the range of 0.5-1.0 % by weight of the overall weight of the composition.

### Lactobionic acid

Lactobionic acid is a polyhydroxy organic acid with humectant and moisturising benefits for the skin. Lactobionic acid is preferably included in an amount within the range of 0.5-2.0 %, more preferably within the range of 0.5-1.0 % by weight of the overall composition.

### Zinc

Zinc is important for skin health and are an essential component of many enzymes and gene transcription factors in the skin. Zinc is preferably included in an amount within the range of 0.05-0.16 %, more preferably 0.08-0.16 % by weight of the overall composition.

The activity of the zinc in the inventive composition is believed to be derived from zink in its ionic Zn²⁺ form dissolved in the water of the composition. Accordingly, any zinc compound which will provide dissolved zinc ions in the product, can be used according to the invention, such as inorganic and organic zinc salts where zinc exists as Zn²⁺. Preferably, a zinc compound containing a skin anion naturally present in the skin, such as, e.g., zinc lactate, zinc pyrrolidone carboxylic acid (Zn-PCA), zinc chloride, zinc stearate, zinc citrate, zinc acetate, zinc adenosine triphosphate, zinc ascorbate, zinc aspartate, zinc carbonate, zinc DNA, zinc glutamate, zinc glycinate, zinc hexametaphosphate, zinc hydrolysed hyaluronate, zinc hydroxide, zinc laurate, zinc linoleate hydroxide, zinc linolenate hydroxide, zinc nitrate, zinc palmitate, and zinc phosphate, is used for providing the zinc.

The inventive topical composition is self-preserving in the absence of a conventional preservative.

Examples of conventional preservatives are benzoic acid, sodium benzoate, ammonium benzoate, calcium benzoate, potassium benzoate, magnesium benzoate, MEA-benzoate, methyl benzoate, ethyl benzoate, propyl benzoate, butyl benzoate, isobutyl benzoate, isopropyl benzoate, phenyl benzoate, propionic acid, ammonium propionate, calcium propionate, magnesium propionate, potassium propionate, sodium propionate, salicylic acid, calcium salicylate, magnesium salicylate, MEA-salicylate, sodium salicylate, potassium salicylate, TEA-salicylate, sorbic acid, calcium sorbate, sodium sorbate, potassium sorbate, formaldehyde, paraformaldehyde, o-phenylphenol, sodium o-phenylphenate, potassium o-phenylphenate, MEA o-phenylphenate, zinc pyrithione, sodium sulfite, ammonium bisulfite, ammonium sulfite, potassium sulfite, potassium hydrogen sulfite, sodium bisulfite, sodium metabisulfite, potassium metabisulfite, chlorobutanol, 4-hydroxybenzoic acid, methylparaben, butylparaben, potassium ethylparaben, potassium paraben, propylparaben, isobutylparaben, sodium methylparaben, sodium ethylparaben, sodium propylparaben, sodium butylparaben, sodium isobutylparaben, ethylparaben, sodium paraben, isopropylparaben, potassium methylparaben, potassium butylparaben, potassium propylparaben, sodium propylparaben, calcium paraben, phenylparaben, dehydroacetic acid, sodium dehydroacetate, formic acid, sodium formate, dibromohexamidine isethionate, thimerosal, phenyl mercuric acetate, phenyl mercuric benzoate, undecylenic acid, potassium undecylenate, sodium undecylenate, calcium undecylenate, TEA-undecylenate, MEA-undecylenate, hexetidine, 5-bromo-5-nitro-1,3-dioxane, 2-bromo-2-nitropropane-1,3-diol, dichlorobenzyl alcohol, triclocarban, p-chloro-m-cresol, triclosan, chloroxylenol, imidazolidinyl urea, polyaminopropyl biguanide, phenoxyethanol, methenamine, quaternium-15, climbazole, DMDM hydantoin, benzyl alcohol, 1-hydroxy-4-methyl-6-(2,4,4-trimethylpentyl) 2-pyridon, piroctone olamine, bromochlorophene, o-cymen-5-ol, isopropyl cresols, methylchloroisothiazolinone, methylisothiazolinone, chlorophene, chloroacetamide, chlorhexidine, chlorhexidine diacetate, chlorhexidine digluconate, chlorhexidine dihydrochloride, phenoxyisopropanol, behentrimonium chloride, cetrimonium bromide, cetrimonium chloride, laurtrimonium bromide, laurtrimonium chloride, steartrimonium bromide, steartrimonium chloride, dimethyl oxazolidine, diazolidinyl urea, hexamidine, hexamidine diisethionate, hexamidine paraben, glutaral, 7-ethylbicyclooxazolidine, chlorphenesin, sodium hydroxymethylglycinate, silver chloride, benzethonium chloride, benzalkonium chloride, benzalkonium bromide, benzalkonium saccharinate, benzylhemiformal, and iodopropynyl butylcarbamate.

Conventionally used preservatives, such as the above-listed, should not be included in the inventive composition, and may not be intentionally added to a composition of the invention. A minor amount, such as in the form of a technically unavoidable impurity, well below an amount known to have an antimicrobial effect could however be tolerated.

In addition to common conventionally used preservatives, other substances having an antimicrobial effect have been widely used, such as polyols like propylene glycol, propanediol, butanediol, butylene glycol, pentylene glycol, hexylene glycol. Such compounds should be absent, except for a technically unavoidable impurity. If however intentionally added for some reason, an amount of any such compounds should be kept very low. Substances with a polar head and a short chain non-polar tail, such as caprylyl glycol, ethylhexylglycerin, and undecylenic acid, caprylyl glycine, glyceryl caprylate, anisic acid, p-anisic acid, phenetyl alcohol and levulinic acid, which may have a cell membrane disturbing effect should preferably also not be included. Especially since the presence of such compound potentially could disturb the skin microbiome and skin barrier function or cause adverse skin reactions.

Other substances than conventional preservatives, such as those listed above, which could have an antimicrobial effect, should preferably not be included in the inventive composition.

Certain essential oils from different parts of plants, like flowers, bark, herbs, wood leaves, seeds, buds, twigs, fruits and roots have also been used for their preservative effect. Plant based products derived from sage, coriander, mustard, clove, oregano, cinnamon, rosemary, turmeric, ginger, cardamom, muscadine, celery, anise, grape, pepper, chives, lemon, pomegranate, cranberry, willow bark, and thyme have also been used for their antimicrobial effect. The main actives of the essential oils and plant-based products are eugenol, thymol, aldehydes, carvacrol, vanillin, allicin, cinnamaldehyde, alkaloids, peptides, citral, saponins, flavonoids, quinones, tannins, coumarins, and caffeic acid.

Other substances than conventional preservatives, such as the above-mentioned other substances having an antimicrobial effect, the essential oils listed above, and the actives listed above of the essential oils, which could have an antimicrobial effect, should preferably not be included in the inventive composition. However, a minor amount, such as up to 3% by weight, preferably no more than 2 %, more preferably up to 1% of the overall composition by weight, could be tolerated for other purposes than preservation and/or the provision of an antimicrobial effect.

The composition also allows the inclusion of various sugar alcohols (polyols), substances that are mainly used for their skin moisturising and hydrating properties but can also serve as nutrients for microorganisms and thus promote growth in the system. Examples of sugar alcohols used are glycerol, fructose, sorbitol, xylitol, mannitol, sucrose and erythritol. A combined amount of these substances must not exceed 20 % by weight of the composition, and should more preferably not exceed 10 %, and more preferably not exceed 5 % by weight of the overall composition.

Examples of topical formulations according to the invention are any one-phase system, or two-phase system containing water, such as solutions, tonics, gels, emulsions, lotions, creams, ointments for use on the skin, including scalp.

The amount of water in the product will typically be dependent on the intended formulation. For example, for an emulsion the water content will typically be within the range of 30-80 % by weight of the formulation, while for a gel, tonic, or solution, the water content will typically be within the range of 60-90 % by weight of the formulation.

Examples of products of the invention are skin care creams, lotions and gels, antiperspirants, deodorants, shaving and after shave products, foundations and concealers and other make-up products, sunscreens, soaps, shower gels and creams, shampoos, and conditioners.

Emulsions are generally preferred according to the invention. The emulsions of the invention can e.g. be o/w or w/o, or multiple emulsions.

Generally, a high water content is preferred according to the invention, such as at least 40 % by weight, more preferably at least 50 % by weight of the formulation.

### Examples

### Example 1

A water in oil emulsion was prepared wherein the actives were dissolved in the water phase.

The below table summarizes formula codes, concentrations of substances used and challenge test method and results achieved.

| **Composition No.** | **Concentrations used of selected substances** | **pH** | **Water content (% w/w)** | **Challenge Test Method** | **Challenge Test Results** |
|---|---|---|---|---|---|
| 1 | 0,16% Zn²⁺ + 0,5% Urea + 1% Lactobionic Acid | 5.6 | 71 | Ph Eur* | Criteria A** |
| 2 | 0,16% Zn²⁺ + 0,5% Urea + 1% Lactobionic Acid | 5.3 | 71 | Ph Eur* | Criteria A** |
| 3 | 0,16% Zn²⁺ + 1% Urea + 1% Lactobionic Acid | 5.3 | 70 | Ph Eur* | Criteria A** |
| 4 | 0,10% Zn²⁺ + 0,7% Urea + 1,0% Lactobionic Acid | 5.5 | 85 | Ph Eur* | Criteria A** |
| 5 | 0,10% Zn²⁺ + 0,7% Urea + 1,0% Lactobionic Acid | 5.5 | 77 | Ph Eur* | Criteria A** |

| | | | | | |
|---|---|---|---|---|---|
| ^{∗} European Pharmacopeia 11.0. 5.1.3 Efficacy of antimicrobial preservation, topical preparations ^{∗∗} Cutaneous application | | | | | |

As can be seen from the above table, all compositions tested fulfilled the A criteria.

## Claims

1. A composition for topical application free of conventional preservatives and having antimicrobial properties, comprising the following components:
0.1-2.0 % by weight of urea;
0.1-2.0 % by weight of lactobionic acid;
0.05-0.2 % by weight of Zn; and,
20-90 % by weight of water
of the overall weight of the composition, provided that the combined amount of Zn, urea, and lactobionic acid is at least 1.1% by weight of the overall weight of the composition.

2. The composition of claim 1, wherein the amount of urea is 0.5-2.0 %, preferably 0.5-1.0 % by weight of the overall weight of the composition.

3. The composition of claim 1 or 2, wherein the amount of lactobionic acid is 0.5-2.0%, preferably 0.5-1.0 % by weight of the overall weight of the composition.

4. The composition of any one of the previous claims, wherein the amount of zinc is 0.08-0.2 %, preferably 0.08-0.16 % by weight of the overall weight of the composition.

5. The composition of any one of the previous claims, wherein the combined amount of Zn, urea, and lactobionic acid is at least 1.8 % by weight of the overall weight of the composition.

6. The composition of any one of the previous claims, wherein the zinc is provided by a zinc salt selected from the group consisting of zinc lactate, zinc pyrrolidone carboxylic acid (Zn-PCA), zinc chloride, zinc stearate, zinc citrate, zinc acetate, zinc adenosine triphosphate, zinc ascorbate, zinc aspartate, zinc carbonate, zinc DNA, zinc glutamate, zinc glycinate, zinc hexametaphosphate, zinc hydrolysed hyaluronate, zinc hydroxide, zinc laurate, zinc linoleate hydroxide, zinc linolenate hydroxide, zinc nitrate, zinc palmitate, and zinc phosphate.

7. The composition of any one of the preceding claims, wherein the composition is in the form of an emulsion.

8. The composition of any one of claims 1 to 6, wherein the composition is in the form of a solution.

9. The composition of any one of claims 1 to 6, wherein the composition is in the form of a gel.

10. The composition of any one of the previous claims, in the form of a cosmetic product.

11. The composition of any one of claims 1-9, in the form of a medical device composition

12. The composition of any one of claims 1-9, in the form of a pharmaceutical composition.
